# EUROPÄISCHE PATENTSCHRIFT

(11) **EP 2 547 120 B1**
(45) Veröffentlichungstag und Bekanntmachung des Hinweises auf die Patenterteilung: **29.05.2013**
(21) Anmeldenummer: 11173647.6
(22) Anmeldetag: 12.07.2011
(51) Int. Cl.: H04R 1/10, H04R 25/00, A61F 11/08

(54) **Vorrichtung und Verfahren zum Prüfen einer Passform einer in einem Gehörgang sitzenden Otoplastik**
Device and method for testing the fit of an otoplastic device in an ear canal
Dispositif et procédé de teste de l'adaptation d'une prothèse auditive à l'intérieur du conduit auditif

(43) Veröffentlichungstag der Anmeldung: 16.01.2013
(73) Patentinhaber: Audio Lab Austria GmbH, 8421 Schwarzau im Schwarzautal (AT)
(72) Erfinder: Thanner, Christoph, 4600 Wels (AT)
(74) Vertreter: Wirnsberger, Gernot

(56) Entgegenhaltungen:
- EP-A1- 1 527 761
- EP-A1- 1 629 808
- AT-A1- 509 033
- US-A1- 2008 011 084
- US-B1- 6 567 524

## Beschreibung

Die Erfindung betrifft eine Vorrichtung zum Prüfen einer Passform einer in einem Gehörgang sitzenden Otoplastik.

Des Weiteren betrifft die Erfindung ein Verfahren zum Prüfen einer Passform einer in einem Gehörgang sitzenden Otoplastik.

Arbeiter in Industriebetrieben sind, neben anderen Umwelteinflüssen, grundsätzlich auch einem großen Lärm ausgesetzt, welcher durch Produktionsanlagen verursacht wird. Um die Arbeiter vor dauerhaften gesundheitlichen Schäden zu schützen, werden verschiedene Lärmschutzsysteme angeboten, die an bzw. in den Ohrmuscheln oder in den Gehörgängen getragen werden.

Unter den bekannten Lärmschutzsystemen sind insbesondere sogenannte Otoplastiken bevorzugt. Otoplastiken weisen menschlichen Ohren bzw. Gehörgängen angepasste äußere Formen bzw. Konturen auf und können in den Ohren bzw. Gehörgängen trotz kleiner Bauweise effektiv einen Schalldurchtritt reduzieren und somit Lärmbeeinträchtigungen verhindern. Diese Otoplastiken sind häufig mit Ventilen ausgestattet, die es erlauben, eine Intensität des durchtretenden Schalls zu regulieren. Alternativ zu Ventilen kommen andere Elemente zur Schallregulierung zum Einsatz, z. B. Steckfilter, die in beispielsweise zentral verlaufende Öffnungen von Otoplastiken eingesetzt werden. Hintergrund für diese konstruktive Maßnahme ist, dass die Otoplastiken einerseits menschliche Ohren vor unerwünscht hoher Lärmbeeinträchtigung schützen sollen, andererseits aber auch sichergestellt sein soll, dass eine gewisse Schalldurchlässigkeit gegeben ist, die beispielsweise eine Kommunikation mit Arbeitskollegen oder insbesondere ein akustisches Erkennen von Warnsignalen, beispielsweise bei Ausfällen von Maschinen im Produktionsbetrieb, erlaubt. Durch die maßgenaue Passform ist ein mehrstündiges Tragen der Otoplastiken möglich.

In der Praxis werden für jeden Benutzer individuell angepasste Otoplastiken z. B. mit voreingestellten Ventilen ausgeliefert. Die individuelle Anpassung erfolgt durch Anfertigen von Abdrücken der Ohren bzw. Gehörgänge des Benutzers und anschließend entsprechende konturgetreue Ausformung der Otoplastiken im Außenbereich. Die Ventile sind ab Werk so eingestellt, dass eine Kommunikation über kurze Distanzen oder ein Erkennen von lauten Warnsignalen möglich ist, Maschinengeräusche jedoch für das menschliche Ohr grundsätzlich unterdrückt werden bzw. meist durch Normen vorgegebene Dämmungswerte erreicht werden. Anschließend werden die Otoplastiken dem Benutzer übergeben. Nach Auslieferung werden die Otoplastiken bei der Arbeit im Betrieb teilweise mehrere Jahre verwendet. Kommen andere schallregulierende Elemente, beispielsweise Steckfilter, zum Einsatz, verhält es sich ähnlich.

Die die Ventile oder allgemein schallregulierenden Elemente umgebenden äußeren und mit einem Ohr bzw. Gehörgang in Kontakt stehenden Teile der Otoplastiken sind in der Regel aus einem Kunststoff gebildet. Obwohl die eingesetzten Kunststoffe durchaus hohe Qualitäten haben und nach langer Tragedauer der Otoplastiken eine zumindest nahezu unveränderte Kontur aufweisen und auch die Ventile sich im Idealfall wie im Werk eingestellt verhalten, kann es doch vorkommen, dass sich die Konturen der menschlichen Ohren bzw. Gehörgänge geringfügig ändern und die Otoplastiken deshalb nicht mehr perfekt sitzen. Daher ist eine Passform der Otoplastiken regelmäßig zu überprüfen, um sicherzustellen, dass auch nach längerer Tragedauer mit den Otoplastiken vorgegebene Dämmungswerte erreicht werden.

Die bekannten Verfahren zur Überprüfung der Passform einer Otoplastik weisen den Nachteil auf, dass die Verfahren äußerst aufwendig sind. Darüber hinaus haftet vielen Verfahren der Nachteil an, dass diese nicht zu genauen und zuverlässigen Ergebnissen führen.

Zur Behebung dieser Nachteile ist gemäß der EP 2 317 773 A2 vorgeschlagen worden, einen Messadapter in eine Otoplastik einzusetzen, wobei der Messadapter zum Zwecke einer Referenzmessung so lose in der Otoplastik befestigt ist, dass Schall zwischen dem Messadapter und der Otoplastik hindurchtreten kann. Für eine Messkurve bzw. ein Messsignal wird der Messadapter in der Otoplastik fest befestigt, sodass kein Schall zwischen dem Messadapter und der Otoplastik hindurchtreten kann. Durch Bildung eines Differenzspektrums kann auf eine Passform bzw. Dichtheit der Otoplastik geschlossen werden. Dieses Verfahren ist grundsätzlich genau, in der Praxis aber relativ umständlich, da am Messadapter hantiert bzw. dieser nach einer Referenzmessung befestigt werden muss. Wird ein Proband mittels eines Kopfhörers beschallt, was der Regelfall ist, kann dies unter Umständen, neben der aufwendigen Messmethodik auch dazu führen, dass sich die Messanordnung geringfügig ändert, wodurch eine Genauigkeit eines Messergebnisses leidet.

Aus der WO 2005/055656 A1 ist eine Messanordnung zur Vermessung einer Otoplastik bekannt geworden, bei welcher ein Messadapter mit zwei Mikrofonen vorgesehen ist, wobei ein erstes außenseitiges Mikrofon zur Aufnahme eines Referenzsignals und ein innenseitiges, dem Trommelfell zugewandtes zweites Mikrofon zur Aufnahme eines Messsignals vorgesehen sind. Die Signale beider Mikrofone werden einer Auswerteeinheit zugeleitet, wobei es jedoch erforderlich ist, die Mikrofone gesondert zu kalibrieren. Darüber hinaus ist die Vorrichtung nicht zur Ermittlung einer Passform einer Otoplastik in einem menschlichen Ohr bzw. Gehörgang bestimmt, da die Otoplastik in einem vorbereitenden Schritt durch Einspritzen eines Polymers perfekt an ein menschliches Ohr bzw. einen Gehörgang angepasst wird.

Des Weiteren zeigt das Dokument US2008/0011084 eine Vorrichtung zum Prüfen einer Passform einer in einem Gehörgang sitzenden Otoplastik, umfassend
- eine Schallerzeugungseinheit;
- einen Messkanal in der Otoplastik;
- einen Messadapter, wobei der Messadapter in der Otoplastik positionierbar ist, sodass Schall in den Messkanal eintreten kann;
- ein Verbindungsmittel, welches den Messkanal mit einem Mikrofon verbindet;
- Schaltungsmittel, um dem Mikrofon Schall aus dem Messkanal zuzuleiten.

Aufgabe der Erfindung ist es, eine Vorrichtung der eingangs genannten Art anzugeben, mit der auf vereinfachte Weise ein Prüfen einer Passform bzw. Dichtheit einer in einem Gehörgang sitzenden Otoplastik möglich ist.

Ein weiteres Ziel der Erfindung ist es, ein Verfahren der eingangs genannten Art anzugeben, das auf einfache Weise ein Prüfen einer Passform einer in einem Gehörgang sitzenden Otoplastik ermöglicht.

Die zuvor genannte Aufgabe wird erfindungsgemäß gelöst, wenn eine Vorrichtung der eingangs genannten Art umfasst:
a) eine Schallerzeugungseinheit;
b) einen Messadapter mit einem Referenzkanal und einem davon getrennten Messkanal, wobei der Messadapter in der Otoplastik positionierbar ist, sodass auf ein der Otoplastik zugewandtes Ende des Messadapters treffender Schall in den Messkanal eintreten kann;
c) zumindest ein Verbindungsmittel, welches den Referenzkanal und den Messkanal mit einem Mikrofon verbindet;
d) Schaltungsmittel, um dem Mikrofon alternativ Schall aus dem Referenzkanal oder dem Messkanal zuzuleiten.

Ein mit der Erfindung erzielter Vorteil ist darin zu sehen, dass eine besonders einfache Vorrichtung zum Prüfen einer Passform bzw. Dichtheit einer in einem Gehörgang sitzenden Otoplastik bereitgestellt wird. Dabei ist von Vorteil, dass der Messadapter sogleich fest in die Otoplastik eingesetzt werden kann und während der Aufnahme eines Referenzspektrums sowie eines Messspektrums positionell nicht mehr verändert werden muss. Dadurch ist ausgeschlossen, dass das Messergebnis durch unsachgemäßes Hantieren verfälscht wird. Darüber hinaus ist die Messung auch genau, da durch Verwendung eines einzigen Mikrofons, dem zur weiteren Auswertung sowohl das Referenzspektrum als auch das Messspektrum alternierend zugeleitet wird, die Bedingungen für die Aufnahme des Messspektrums im Wesentlichen ident mit den Bedingungen für die Aufnahme des Referenzspektrums sind, was ebenfalls einer Qualität des Messergebnisses zugutekommt. Des Weiteren ist auch die Aufnahme eines Referenzspektrums einfach und insbesondere keine Kalibrierung von Mikrofonen bzw. Abstimmung derselben erforderlich.

Das Schaltungsmittel kann an sich beliebig ausgebildet sein, um alternativ Schall aus dem Referenzkanal oder dem Messkanal zu einem einzelnen Mikrofon zuzuleiten. Bevorzugt ist jedoch das Schaltungsmittel als Ventil ausgebildet, um ein rasches Umschalten zu ermöglichen. Insbesondere kann vorgesehen sein, dass das Ventil als 3/2-Wege-Ventil ausgebildet ist.

Des Weiteren kann ein Kopfhörer vorgesehen sein. Ein Beschallen des in die Otoplastik eingesetzten Messadapters kann dann unmittelbar über den Kopfhörer erfolgen. Möglich ist es auch, dass die Schallerzeugungseinheit innerhalb des Kopfhörers angeordnet ist, um den Messadapter unmittelbar zu beschallen.

Das Mikrofon ist vom Messadapter beabstandet angeordnet. Dabei kann das Mikrofon insbesondere ortsfest beabstandet zum Messadapter angeordnet sein, z. B. in einer Auswerteeinheit. Allfällige Bewegungen eines Probanden während einer Messung stören dann grundsätzlich nicht. Im Unterschied zu einer Freifeldmessung bleibt ein Abstand zwischen Signalquelle bzw. Schallerzeugungseinheit, dem Ohr, der Otoplastik und dem Messadapter gleich, weshalb die Messergebnisse genauer sind. Sofern ein Kopfhörer vorgesehen ist, führt zumindest ein Verbindungsmittel vom Messadapter zwischen einem Polster des Kopfhörers und dem Kopf eines Probanden zum Mikrofon.

Das zumindest eine Verbindungsmittel ist bevorzugt in Form von zwei Schläuchen ausgebildet. Die Schläuche bestehen typischerweise aus einem Silicon oder Kunststoff und weisen damit eine ausreichende Flexibilität bzw. Biegsamkeit auf, sodass der Messadapter einfach mit dem Mikrofon verbunden werden kann.

Grundsätzlich ist zumindest ein Verbindungsmittel ausreichend, um den Messadapter mit dem Mikrofon zu verbinden, wenn das Verbindungsmittel an einer Stelle so verzweigt ist, dass dieses einerseits mit dem Referenzkanal und andererseits mit dem Messkanal in Verbindung steht. Bevorzugt ist es jedoch, dass der Messadapter zwei Anschlüsse für zwei Verbindungsmittel aufweist, wobei ein Anschluss mit dem Referenzkanal und ein Anschluss mit dem Messkanal in Verbindung steht. Eine Zuleitung des Referenzspektrums sowie des Messspektrums bzw. Schall zum Mikrofon kann dann durch das vorgesehene Schaltungsmittel gesteuert werden.

Der Messadapter kann einteilig oder auch mehrteilig ausgebildet sein. Bei einer mehrteiligen Ausbildung umfasst der Messadapter zwei Teile, wobei ein erstes Teil die Anschlüsse umfasst und ein zweites Teil das in der Otoplastik positionierbare Endstück ist. Eine mehrteilige Variante bietet insbesondere Vorteile, wenn die Otoplastik ein Gewinde aufweist, in welches üblicherweise ein Ventil eingeschraubt ist. Bei einer mehrteiligen Ausbildung des Messadapters kann dann das Endstück, das ebenfalls mit einem passenden Gewinde ausgebildet ist, eingeschraubt werden. Anschließend wird der erste Teil des Messadapters aufgesetzt. Durch diese Vorgehensweise bzw. die mehrteilige Ausbildung wird vermieden, dass bei einem Einschrauben des Messadapters eine Verdrillung der Verbindungsmittel bzw. Schläuche auftritt. Möglich ist es aber auch, dass der Messadapter oder ein Teil desselben eingesteckt, aufgeklickt oder mittels einer Halbdrehung an und/oder in der Otoplastik befestigt wird. Die Ausbildung des Messadapters in einteiliger oder mehrteiliger Form bzw. die Verwendung eines bestimmten Messadapters hängt auch davon ab, ob die Otoplastik aus einem harten oder weichen Material gefertigt ist.

Der Messadapter ist üblicherweise länglich ausgebildet und weist eine erste Öffnung auf, die ein erstes Ende des Referenzkanals definiert. Des Weiteren kann der Messadapter eine zweite Öffnung aufweisen, die endseitig angeordnet ist und einen Einlass des Messkanals definiert.

Das verfahrensmäßige Ziel der Erfindung wird erreicht durch ein Verfahren zum Prüfen einer Passform einer in einem Gehörgang sitzenden Otoplastik, die einen Kanal aufweist, der einen trommelfellseitigen Bereich der Otoplastik mit einem außenseitigen Bereich der Otoplastik verbindet, wobei ein Messadapter mit einem Referenzkanal und einem davon getrennten Messkanal in und/oder an der Otoplastik positioniert wird, sodass der Messkanal mit dem Kanal in Verbindung steht, wonach eine Beschallung erfolgt und Schall aus dem Referenzkanal und dem Messkanal abwechselnd einer Auswerteeinheit zugeleitet wird.

Ein mit einem erfindungsgemäßen Verfahren erzielter Vorteil ist darin zu sehen, dass auf einfache Weise und ohne weitere Kalibrierung von Mikrofonen eine Dichtheit einer Otoplastik bzw. deren Passform in einem menschlichen Gehörgang überprüft werden kann.

Bevorzugt ist vorgesehen, dass mit der Auswerteeinheit, insbesondere einem Mikrofon, eine Differenz eines Messspektrums abzüglich eines Referenzspektrums gebildet wird. Durch diese Differenzbildung kann auf die Dichtheit der Otoplastik bzw. deren Passform im menschlichen Gehörgang geschlossen werden.

Grundsätzlich ist es möglich, die Messung in verschiedenen Bereichen des menschlichen Hörspektrums durchzuführen. Zweckmäßig und ausreichend ist es jedoch, dass die Beschallung bei einer Frequenz von weniger als 300 Hz durchgeführt wird.

Um Umgebungseinflüsse möglichst auszuschalten, wird das Beschallen bevorzugt mit einem Kopfhörer oder innerhalb eines Kopfhörers durchgeführt.

Bevorzugt ist es ferner, wenn die Auswerteeinheit ein Mikrofon umfasst, das beabstandet zum Messadapter angeordnet wird, da dann Bewegungen eines Probanden während einer Messung nicht störend wirken.

Weitere Merkmale, Vorteile und Wirkungen der Erfindung ergeben sich anhand des nachfolgend dargestellten Ausführungsbeispiels. In den Zeichnungen, auf welche dabei Bezug genommen wird, zeigen:
Fig. 1 mehrere Teile eines Messadapters;
Fig. 2 einen Messadapter gemäß Fig. 1 nach Verbinden der einzelnen Teile;
Fig. 3 eine schematische Darstellung einer Messanordnung.

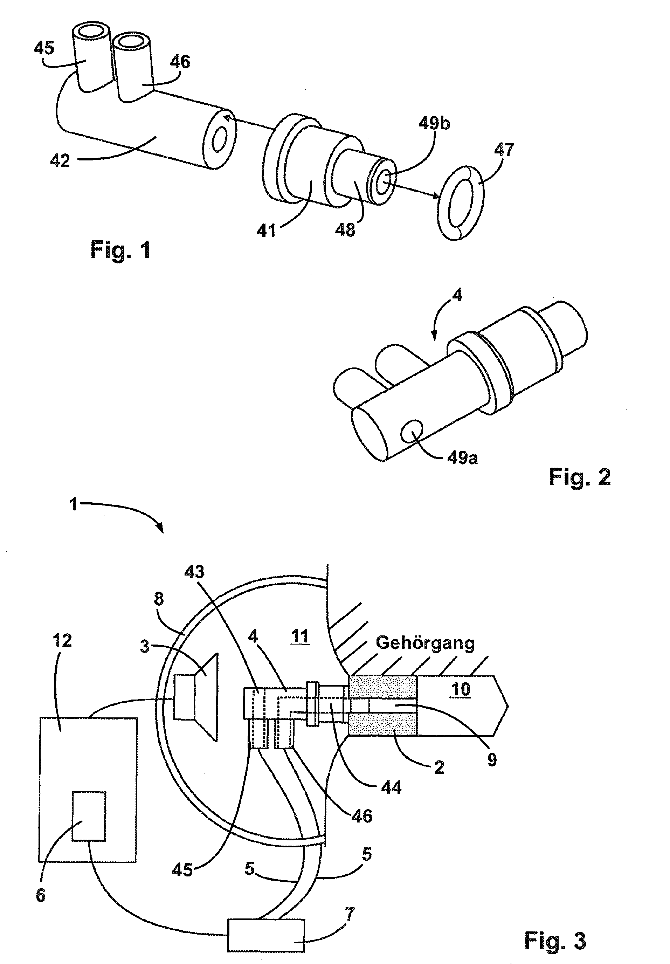

In Fig. 1 ist ein Messadapter 4, der aus mehreren Teilen besteht, schematisch dargestellt. Der Messadapter 4 weist ein erstes Teil 42 mit zwei Anschlüssen 45, 46 und ein zweites Teil, das als ein gesondertes Endstück 41 ausgebildet ist, auf. Eine einteilige Ausbildung des Messadapters 4 ist ebenfalls möglich. Des Weiteren ist eine ringförmige Dichtung 47 vorgesehen. Das erste Teil 42 ist mit einem zylindrischen Grundkörper ausgebildet, an dem an einem Ende umfangsseitig die Anschlüsse 45, 46 angeordnet sind. An dem anderen Ende des Grundkörpers ist das erste Teil 42 mit einer zylindrischen Umfangsfläche ausgebildet. Das erste Teil 42 ist in diesem Bereich mit einem äußeren Durchmesser ausgebildet, der ein Einsetzen des ersten Teils 42 in das Endstück 41 erlaubt, das innen eine entsprechend ausgebildete Aufnahme aufweist, wobei gegebenenfalls auch eine innen im Endstück 41 anliegende Dichtung vorgesehen sein kann, um das erste Teil 42 mit dem Endstück 41 schalldicht zu verbinden. Im zusammengebauten Zustand gemäß Fig. 2 ist das erste Teil 42 in das Endstück 41 eingesetzt und die Dichtung 47 an einem verjüngten Ende 48 am Endstück 41 aufgesetzt. Das Endstück 41 kann, auch bei einteiliger Ausbildung des Messadapters 4, am verjüngten Ende 48 mit einem Gewinde ausgebildet sein, damit der Messadapter 4 in eine Otoplastik 2 einschraubbar ist, wenn diese ein Gewinde aufweist.

Wie aus Fig. 2 ersichtlich ist, weist das erste Teil 42 eine erste Öffnung 49a auf. Diese erste Öffnung 49a bildet einen Eingang für einen Referenzkanal 43, der sich von dieser ersten Öffnung 49a normal zur Längsachse des ersten Teils 42 verlaufend bis hin zum Auslass des Anschlusses 45 erstreckt. Des Weiteren weist der erste Teil 42 eine zweite Öffnung 49b bzw. Bohrung auf, in welcher ein Messkanal 44 zum Auslass des Anschlusses 46 verläuft. Im Inneren des ersten Teils 42 ist der Messkanal 44 vom Referenzkanal 43 getrennt, der von der ersten Öffnung 49a zum Auslass des Anschlusses 45 verläuft. Das Endstück 41 ist, wie in Fig. 1 ersichtlich, ebenfalls mit einer Öffnung bzw. einem Kanal ausgebildet, sodass bei der zusammengebauten Darstellung gemäß Fig. 2 in das Endstück 41 eintretender Schall schließlich am Auslass des Anschlusses 46 austritt.

Anhand von Fig. 3 ist eine erfindungsgemäße Vorrichtung 1 und deren Anwendung näher erläutert. Die Vorrichtung 1 wird verwendet, um eine Dichtheit einer Otoplastik 2 in einem menschlichen Gehörgang zu prüfen. Dies ist insbesondere bei einer Otoplastik 2 erforderlich, die Lärm vom menschlichen Ohr abhalten oder zumindest auf ein bestimmtes Maß reduzieren soll. Bei längerer Verwendung einer Otoplastik 2 kann es dazu kommen, dass diese einem bestimmten Verschleiß unterliegt und daher nicht mehr optimal sitzt. Darüber hinaus ist es auch möglich, dass die Otoplastik 2 nicht gut anliegt, da sich ein Gehörgang im Laufe der Zeit geringfügig geändert hat. Bei der Otoplastik 2 handelt es sich üblicherweise um eine solche, die mit einem herausnehmbaren Ventil ausgestattet ist, das beispielsweise in die Otoplastik 2 eingeschraubt sein kann, wofür die Otoplastik 2 mit einem innenseitigen Gewinde versehen ist. Ein Aufbau einer derartigen Otoplastik 2 ist beispielsweise in der EP 2 317 773 A2 beschrieben.

Die Vorrichtung 1 umfasst den Messadapter 4 mit den Anschlüssen 45, 46 und dem innenliegenden Referenzkanal 43 und dem davon getrennten Messkanal 44. Des Weiteren ist eine Schallerzeugungseinheit 3 vorgesehen, mit welcher der Messadapter 4 bzw. die Otoplastik 2 beschallt werden kann. Wie dargestellt, kann für die Zwecke einer Messung auch ein Kopfhörer 8 vorgesehen sein. Gegebenenfalls ist es auch möglich, dass die Schallerzeugungseinheit 3 außerhalb des Kopfhörers 8 angeordnet und Schall über den Kopfhörer 8 auf den Messadapter 4 bzw. die Otoplastik 2 aufgebracht wird. Darüber hinaus sind Verbindungsmittel 5 vorgesehen, welche den Anschluss 45 sowie den Anschluss 46 jeweils mit Eingängen eines Schaltungsmittels 7, beispielsweise eines 3/2-Wege-Ventils, verbindet. Dem Schaltungsmittel 7 ist ein Mikrofon 6 nachgeschaltet, sodass je nach Schaltung des Ventils dem Mikrofon 6 alternativ Schall aus dem Referenzkanal 43 oder dem Messkanal 44 zuführbar ist. Das Mikrofon 6 kann Teil einer Einheit 12 sein, welche auch einen Computer umfasst. Über diese Einheit 12 kann auch die Schallerzeugungseinheit 3 angesteuert werden, sodass sich insgesamt ein kompakter Aufbau ergibt.

Im Betrieb bzw. bei Anwendung der Vorrichtung 1 wird die Dichtigkeit bzw. Passform einer Otoplastik 2 in einem menschlichen Gehörgang überprüft, in dem zunächst aus einem Kanal 9 der Otoplastik 2 ein Ventil oder gegebenenfalls ein anderes schallregulierendes Element entnommen wird, sodass der Kanal 9 frei ist. Anschließend wird der Messadapter 4 in der Otoplastik 2 befestigt, und zwar so, dass das verjüngte Ende 48 in der Otoplastik 2 eingeführt wird. Sofern die Otoplastik 2 endseitig mit einem Gewinde ausgebildet ist, in dem üblicherweise ein Ventil eingeschraubt ist, und der Messadapter 4 bzw. dessen Endstück 41 ebenfalls ein Gewinde aufweist, kann das Endstück 41 bei mehrteiligem Aufbau des Messadapters 4 vorerst alleine in die Otoplastik 2 eingeschraubt werden. In diesem Fall wird anschließend das erste Teil 42 auf das bereits befestigte Endstück 41 aufgesetzt bzw. eingeschoben. Die Dichtung 47 sorgt bei der Befestigung für eine ausreichende Schalldämmung. Anschließend wird von einem Probanden der Kopfhörer 8 aufgesetzt und es erfolgt ein Beschallen mit einem Signal von 250 Hz. Dabei wird ein Referenzspektrum aufgenommen, wobei das Ventil so geschaltet ist, dass in den Referenzkanal 43 eintretender Schall zum Mikrofon 6 geleitet wird. Filter, die mögliche Artefakte erkennen und/oder ausfiltern können (z. B. eine Schluckbewegung des Probanden), werden dabei auf null gesetzt. Anschließend wird bei unveränderter Messsituation das Ventil geschaltet, sodass Schall aus dem Messkanal 44 zum Mikrofon 6 gelangt. Ist die Otoplastik 2 undicht, so gelangt Schall zwischen der Otoplastik 2 und dem menschlichen Gehörgang in den Kanal 9, der einen trommelfellseitigen Bereich 10 der Otoplastik 2 mit einem außenseitigen Bereich 11 der Otoplastik 2 verbindet, und damit in den Messkanal 44 und von dort über den Auslass des Anschlusses 46 und das Verbindungsmittel 5 letztlich zum Mikrofon 6. Eine Bewegung eines Probanden bei einer Messung stört dann nicht, weil das Mikrofon 6 sowie die relative Anordnung von Otoplastik 2 und Messadapter 4 dadurch nicht beeinflusst wird. Durch Bildung eines Differenzspektrums kann ermittelt werden, ob und wenn in welchem Ausmaß die Otoplastik 2 nicht mehr perfekt im Gehörgang anliegt. Selbstverständlich ist es auch möglich, zuerst das Messspektrum und anschließend das Referenzspektrum aufzunehmen oder mehrere Messungen zum Zwecke einer Mittelwertbildung bzw. statistischen Absicherung durchzuführen.

Es versteht sich, dass zwei idente Vorrichtungen 1 bei einer Messung eingesetzt werden können, wenn zwei Otoplastiken 2 in einem Messdurchgang überprüft werden sollen, was der Regelfall ist. Allerdings ist in diesem Fall nur eine Einheit 12 mit zwei Mikrofonen 6 erforderlich.

## Patentansprüche

1. Vorrichtung (1) zum Prüfen einer Passform einer in einem Gehörgang sitzenden Otoplastik (2), umfassend
a) eine Schallerzeugungseinheit (3);
b) einen Messadapter (4) mit einem Referenzkanal (43) und einem davon getrennten Messkanal (44), wobei der Messadapter (4) in der Otoplastik (2) positionierbar ist, sodass auf ein der Otoplastik (2) zugewandtes Ende des Messadapters (4) treffender Schall in den Messkanal (44) eintreten kann;
c) zumindest ein Verbindungsmittel (5), welches den Referenzkanal (43) und den Messkanal (44) mit einem Mikrofon (6) verbindet;
d) Schaltungsmittel (7), um dem Mikrofon (6) alternativ Schall aus dem Referenzkanal (43) oder dem Messkanal (44) zuzuleiten.

2. Vorrichtung (1) nach Anspruch 1, wobei das Schaltungsmittel (7) als Ventil ausgebildet ist.

3. Vorrichtung (1) nach Anspruch 2, wobei das Ventil als 3/2-Wege-Ventil ausgebildet ist.

4. Vorrichtung (1) nach einem der Ansprüche 1 bis 3, wobei ein Kopfhörer (8) vorgesehen ist.

5. Vorrichtung (1) nach einem der Ansprüche 1 bis 4, wobei das Mikrofon (6) vom Messadapter (4) beabstandet angeordnet ist.

6. Vorrichtung (1) nach einem der Ansprüche 1 bis 5, wobei das zumindest eine Verbindungsmittel (5) in Form von zwei Schläuchen ausgebildet ist.

7. Vorrichtung (1) nach einem der Ansprüche 1 bis 6, wobei der Messadapter (4) zwei Anschlüsse (45, 46) für zwei Verbindungsmittel (5) aufweist, wobei ein Anschluss (45) mit dem Referenzkanal (43) und ein Anschluss (46) mit dem Messkanal (44) in Verbindung steht.

8. Vorrichtung (1) nach Anspruch 7, wobei der Messadapter (4) mehrteilig ausgebildet ist, wobei ein erstes Teil (42) die Anschlüsse (45, 46) umfasst, und ein zweites Teil das ein in der Otoplastik (2) positionierbares Endstück ist.

9. Vorrichtung (1) nach einem der Ansprüche 1 bis 8, wobei der Messadapter (4) länglich ausgebildet ist und eine erste Öffnung (49a) aufweist, die ein erstes Ende des Referenzkanals (43) definiert.

10. Vorrichtung (1) nach Anspruch 9, wobei der Messadapter (4) eine zweite Öffnung (49b) aufweist, die endseitig angeordnet ist und einen Einlass des Messkanals (44) definiert.

11. Verfahren zum Prüfen einer Passform einer in einem Gehörgang sitzenden Otoplastik (2), die einen Kanal (9) aufweist, der einen trommelfellseitigen Bereich (10) der Otoplastik (2) mit einem außenseitigen Bereich (11) der Otoplastik (2) verbindet, wobei ein Messadapter (4) mit einem Referenzkanal (43) und einem davon getrennten Messkanal (44) in und/oder an der Otoplastik (2) positioniert wird, sodass der Messkanal (44) mit dem Kanal (9) in Verbindung steht, wonach eine Beschallung erfolgt und Schall aus dem Referenzkanal (43) und dem Messkanal (44) abwechselnd einer Auswerteeinheit zugeleitet wird.

12. Verfahren nach Anspruch 11, wobei eine Differenz eines Messspektrums abzüglich eines Referenzspektrums gebildet wird.

13. Verfahren nach Anspruch 11 oder 12, wobei die Beschallung bei einer Frequenz von weniger als 300 Hz durchgeführt wird.

14. Verfahren nach einem der Ansprüche 11 bis 13, wobei das Beschallen mit einem Kopfhörer (8) oder innerhalb eines Kopfhörers (8) durchgeführt wird.

15. Verfahren nach einem der Ansprüche 11 bis 14, wobei die Auswerteeinheit ein Mikrofon (6) umfasst, das beabstandet zum Messadapter angeordnet wird.

## Claims

1. A device (1) for testing the fit of an ear mould (2) sitting in an auditory canal, comprising
a) a sound-generation unit (3);
b) a measuring adapter (4) with a reference channel (43) and a measuring channel (44) separate therefrom, wherein the measuring adapter (4) can be positioned in the ear mould (2), so that sound encountering an end of the test adapter (4) turned towards the ear mould (2) is able to enter the test channel (44);
c) at least one connecting means (5), which connects the reference channel (43) and the measuring channel (44) to a microphone (6);
d) switching means (7) for alternatively conducting sound from the reference channel (43) or the measuring channel (44) to the microphone (6).

2. The device (1) according to claim 1, wherein the switching means (7) is configured as a valve.

3. The device (1) according to claim 2, wherein the valve is configured as a 3/2-way valve.

4. The device (1) according to one of the claims 1 to 3, wherein a headset (8) is provided.

5. The device (1) according to one of the claims 1 to 4, wherein the microphone (6) is spaced apart from the measuring adapter (4).

6. The device (1) according to one of the claims 1 to 5, wherein the at least one connecting means (5) is configured in the form of two tubes.

7. The device (1) according to one of the claims 1 to 6, wherein the measuring adapter (4) comprises two connections (45, 46) for two connecting means (5), wherein one connection (45) is connected to the reference channel (43) and one connection (46) is connected to the measuring channel (44).

8. The device (1) according to claim 7, wherein the measuring adapter (4) is multi-part in design, wherein a first part (42) comprises the connections (45, 46) and a second part is the one end piece which can be positioned in the ear mould (2).

9. The device (1) according to one of the claims 1 to 8, wherein the measuring adapter (4) is elongated in design and comprises a first opening (49a) which defines a first end of the reference channel (43).

10. The device (1) according to claim 9, wherein the measuring adapter (4) exhibits a second opening (49b) which is arranged at the end and defines an inlet of the measuring canal (44).

11. A method of testing the fit of an ear mould (2) sitting in an auditory canal, comprising a channel (9) which connects a region (10) on the ear-drum side of the ear mould (2) to a region (11) on the outside of the ear mould (2), wherein a measuring adapter (4) with a reference channel (43) and a measuring channel (44) separate therefrom is positioned in and/or on the ear mould (2), so that the measuring channel (44) is connected to the channel (9), according to which ultrasonic exposure takes place and sound from the reference channel (43) and the measuring channel (44) is alternately supplied to an evaluation unit.

12. The method according to claim 11, wherein a difference of a measuring spectrum less a reference spectrum is created.

13. The method according to claim 11 or 12, wherein the ultrasonic exposure is carried out at a frequency of under 300 Hz.

14. The method according to one of the claims 11 to 13, wherein the ultrasonic exposure is carried out with a headset (8) or within a headset (8).

15. The method according to one of the claims 11 to 14, wherein the evaluation unit comprises a microphone (6), which is spaced apart from the measuring adapter.

## Revendications

1. Dispositif (1) destiné à vérifier une forme d'adaptation d'un moulage otoplastique (2) située dans un canal auditif, comprenant
a) un système générateur de sons (3);
b) un adaptateur de mesure (4) avec un canal de référence (43) et un canal de mesure (44) séparé de celui-ci, l'adaptateur de mesure (4) étant positionnable dans le moulage otoplastique (2) de sorte que sur une extrémité de l'adaptateur de mesure (4) orientée vers le moulage otoplastique (2) un son approprié peut entrer dans le canal de mesure (44),
c) au moins un moyen de liaison (5) qui relie le canal de référence (43) et le canal de mesure (44) avec un microphone (6),
d) un moyen de connexion (7) pour transmettre alternativement au microphone (6), un son venant du canal de référence (43) ou du canal de mesure (44).

2. Dispositif (1) selon la revendication 1 le moyen de connexion (7) étant constitué comme une vanne.

3. Dispositif (1) selon la revendication 2, la vanne étant constituée comme une vanne à 3/2 voies.

4. Dispositif (1) selon une quelconque des revendications 1 à 3 pour lequel un écouteur (8) est prévu.

5. Dispositif (1) selon une quelconque des revendications 1 à 4, le microphone (6) étant disposé à distance de l'adaptateur de mesure (4).

6. Dispositif (1) selon une quelconque des revendications 1 à 5, le au moins un moyen de liaison (5) étant constitué sous la forme de deux flexibles.

7. Dispositif (1) selon une quelconque des revendications 1 à 6, l'adaptateur de mesure (4) présentant deux raccords (45, 46) pour deux moyens de liaison (5), pour lequel un raccord (45) avec le canal de référence (43) et un raccord (46) avec le canal de mesure (44) sont en liaison.

8. Dispositif (1) selon la revendication 7, l'adaptateur de mesure (4) étant constitué en plusieurs parties, une première partie (42) comprenant les raccords (45, 46) et une deuxième partie étant la pièce d'extrémité positionnable dans le moulage otoplastique (2).

9. Dispositif (1) selon une quelconque des revendications 1 à 8, l'adaptateur de mesure (4) étant constitué de façon allongée et présentant une première ouverture (49a) qui définit une première extrémité du canal de référence (43).

10. Dispositif (1) selon la revendication 9, l'adaptateur de mesure (4) présentant une deuxième ouverture (49b) qui est disposée en extrémité et définit une entrée du canal de mesure (44).

11. Procédé destiné à contrôler une forme d'adaptation d'un moulage otoplastique (2) située dans un canal auditif qui présente un canal (9) qui relie une zone (10) du moulage otoplastique (2) située du côté de la membrane du tympan avec une zone (11) du moulage otoplastique (2) située en extérieur, un adaptateur de mesure (4) avec un canal de référence (43) et un conduit de mesure (44) séparé de celui-ci étant positionné dans et/ou sur le moulage otoplastique (2) de sorte que le canal de mesure (44) est en liaison avec le canal (9), d'après quoi il se produit une sonorisation et un son est acheminé depuis le canal de référence (43 et le canal de mesure (44), alternativement d'un système d'évaluation.

12. Procédé selon la revendication 11, une différence étant constituée par déduction d'un spectre de référence d'un spectre de mesure.

13. Procédé selon la revendication 11 ou 12, la sonorisation étant réalisée à une fréquence inférieure à 300 Hz.

14. Procédé selon une quelconque des revendications 11 à 13, la sonorisation étant réalisée avec un écouteur (8) ou à l'intérieur d'un écouteur (8).

15. Procédé selon une quelconque des revendications 11 à 14, le système d'évaluation comprenant un microphone (6) qui est disposé distant de l'adaptateur de mesure.
